(19) **Europäisches Patentamt / European Patent Office / Office européen des brevets**

(11) **EP 4 552 548 A1**

(12) **EUROPEAN PATENT APPLICATION**
published in accordance with Art. 153(4) EPC

(43) Date of publication:
**14.05.2025 Bulletin 2025/20**

(21) Application number: **23835274.4**

(22) Date of filing: **19.06.2023**

(51) International Patent Classification (IPC):
**A61B 1/045** (2006.01)    **G06T 1/00** (2006.01)

(52) Cooperative Patent Classification (CPC):
**A61B 1/045; G06T 1/00**

(86) International application number:
**PCT/JP2023/022550**

(87) International publication number:
**WO 2024/009740 (11.01.2024 Gazette 2024/02)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.07.2022 JP 2022110099**

(71) Applicant: **National Institute of Advanced
Industrial
Science and Technology
Chiyoda-ku
Tokyo 100-8921 (JP)**

(72) Inventors:
- **NOSATO Hirokazu**
  **Tsukuba-shi, Ibaraki (JP)**
- **SAKANASHI Hidenori**
  **Tsukuba-shi, Ibaraki (JP)**
- **IWAKI Takuya**
  **Tsukuba-shi, Ibaraki (JP)**

(74) Representative: **Wilson Gunn
Centurion House
129 Deansgate
Manchester M3 3WR (GB)**

(54) **METHOD AND SYSTEM FOR PRODUCING LEARNING IMAGE DATA**

(57)    The present invention is to provide a learning image data creating method and system therefor that can be created by learning image data of learning models without being affected by the distortions of the portions at the periphery of the endoscopic images. The present invention performs a mask extension process that is performed to make a region where the mucosal image is distorted, due to the influence of a lens of an endoscope which is adjacent to the mask image and observes mucosal, into an extended region image of the mask image to create extension processed learning image data. The present invention performs color conversion correction on the mask image and the extended region image of the extension processed learning image data to create mask color replaced learning image data, to correct the color tone of the mucosal image as the difference in the color tone of the mucosal image in the mask color replaced learning image data is not affected to create mucosal color tone corrected learning image data, and to use the mucosal color tone corrected learning image data as the learning image data.

EP 4 552 548 A1

## Fig. 2

```
┌─────────────────────────────────────┐
│           Processing flow            │
└─────────────────────────────────────┘
                  │
┌─────────────────────────────────────┐
│      A. Endoscopic image            │──── ST1
└─────────────────────────────────────┘
                  │
┌─────────────────────────────────────┐
│   ① Mask extension process          │──── ST2
└─────────────────────────────────────┘
                  │
┌─────────────────────────────────────┐
│   B. Mask extension-processed        │──── ST3
│      learning image data storage     │
└─────────────────────────────────────┘
                  │
┌─────────────────────────────────────┐
│  ② Mask region color replaced process│──── ST4
└─────────────────────────────────────┘
                  │
┌─────────────────────────────────────┐
│   C. Mask color replaced             │──── ST5
│      learning image data storage     │
└─────────────────────────────────────┘
                  │
┌─────────────────────────────────────┐
│  ③ Mucosal color tone correction     │──── ST6
│     process                          │
└─────────────────────────────────────┘
                  │
┌─────────────────────────────────────┐
│  D. Mucosal color tone corrected     │──── ST7
│     learning image data storage      │
└─────────────────────────────────────┘
                  │
┌─────────────────────────────────────┐
│  ④ Image data expansion process      │──── ST8
└─────────────────────────────────────┘
                  │                        ST9
┌─────────────────────────────────────┐
│  E. Data expanded                    │──▷ Artificial intelli-
│     learning image data storage      │    -gence learning
└─────────────────────────────────────┘    etc.
```

**Description**

TECHNICAL FIELD

The present invention relates to a learning image data creating method and system for creating learning image data by using an endoscopic image as learning image data.

BACKGROUND FIELD

For example, Hunner-type interstitial cystitis is an inflammatory disease characterized in that the presence of specific red mucosal lesions called Hunner lesions is recognized in a bladder. However, there are no objective diagnostic indicators for Hunner lesions, and the diagnosis of Hunner lesions would be made subjectively by the decision of the cystoscopist. Therefore, the discrimination between similar red mucosal lesions seen in BCG-related cystitis or carcinoma in situ and genuine red mucosal lesions merely based on the observations of cystoscopic images is difficult even experienced doctors who specialize in interstitial cystitis. In order to discriminate lesions in which the characteristics of mucosal are similar as described above, it will be important to improve the accuracy of lesion discrimination in the endoscopic inspection, and it will be also necessary to improve the diagnostic accuracy with assisting diagnosis of the doctors supported by using artificial intelligence or the like.

Conventionally, as artificial intelligence for supporting diagnosis, there have been some technologies that create learning models by learning endoscopic images as learning image data. For example, in Japanese Patent Publication 2020-141995 (Patent Document 1), a technology that includes to generate superimposed images which superimpose a foreground endoscopic image with an endoscopic treatment tool extracted and a background endoscopic image which is the background of the foreground endoscopic image, to create a large amount of learning image data for image recognition by using the superimposed images and to create learning models by using the learning image data.

In addition, as a technology that identifies the observation direction of the mucosal of which mucosal images observed, Japanese Patent 3,720,617 (Patent Document 2) discloses to add indicators on mask images in order to identify the direction of the endoscopic images.

Related Art Documents

Patent Documents

Patent Document 1: Japanese Patent Publication 2020-141995 (September 10, 2020)
Patent Document 2: Japanese Patent 3,720,617 (November 30, 2005)

SUMMARY OF INVENTION

TECHNICAL PROBLEM

In conventional technologies, there have been no recognitions that the distortion of mucosal images at the periphery of endoscopic images due to the influence of a lens of an endoscopic scope which observes the mucosal or color differences of the boundary between the mucosal images and mask images (the differences of hue, saturation and value) may affect learning results of artificial intelligence in endoscopic images. For the above-mentioned reason, conventional technologies have created learning image data by using endoscopic image data themselves in which the mask images were positioned around the mucosal images. Therefore, the quality of the learning image data was poor even if using expanded image data that was created by using an expanded technology of learning image data, as a result, there was a limitation to improve the accuracy of learning models. For example, in the cases of Hunner-type interstitial cystitis there have been very few cases, since the quality of learning data cannot be covered by the quantity of learning data, the fact described above will lead to a problem that directly affects the learning accuracy of the learning models.

An object of the present invention is to provide a learning image data creating method and system therefor that can be created by learning image data of learning models without being affected by the distortions of the portions at the periphery of the endoscopic images and the color differences of mucosal image and mask image.

SOLUTION TO PROBLEM

The present invention relates to learning image data creating method for creating learning image data used to create a learning model, based on an endoscopic image in which a mask image exists outside a mucosal image. The method of the present invention comprises creating extension processed learning image data that has an extended region image of the mask image which is made by a mask extension process that defines the extended region image with a region which is adjacent to the mask image and where the mucosal image is distorted due to the influence of a lens of an endoscope for observing mucosal. In addition, it is preferable to comprise creating mask color replaced learning image data by performing color conversion correction on the mask image and the extended region image of the extension processed learning image data, creating mucosal color tone corrected learning image data performing by the color tone correction of the mucosal image as the difference in the color tone of the mucosal image in the mask color replaced learning image data is not affected and defining the mucosal color tone corrected learning image data as the learning image data. It is also preferable that the learning data on which the expansion is based contain as few noise elements as possible when there is not enough learning data for endoscopic images. As a result of various studies based on such a premise mentioned above, the inventors of the present invention have found that the mucosal image adjacent to the mask image may be distorted due to the influence of a lens or the like. The inventors then, based on the above-confirmed knowledge, have created extension processed learning image data that has an extended region image of the mask image which is made by a mask extension process that defines the extended region image with a region which is adjacent to the mask image and where the mucosal image is distorted due to the influence of a lens of an endoscope for observing mucosal. The inventors of the present invention have replaced the mask image and the extended region image with pattern images of the color in which the distortion of mucosal images is not caused by creating mask color replaced learning image data by performing color conversion correction on the mask image and the extended region image of the extension processed learning image data. Next, the inventors of the present invention have created mucosal color tone corrected learning image data by performing the color tone correction of the mucosal image as the difference in the color tone of the mucosal image in the mask color replaced learning image data is not affected for the learning result. The color tone difference of images can be corrected by the color tone correction corresponding to the type of endoscopes to be used. Note that the color tone correction can be performed before the above-mentioned color conversion correction. The learning image data contains almost no noise elements when the conversion correction and the color tone correction are performed so that the learning accuracy of the learning model can be improved.

Note that the method of the present invention may create expanded learning image data by image expansion technologies, using the mucosal color tone corrected learning image data, and may define the expanded learning image data as the learning image data. Thereby, even if the quality of learning image data is not supported by the quantity of learning image data, the learning accuracy of the learning model can be improved.

Normal mask image includes an indicator that confirms the up and down direction of the endoscopic image. In this case, it is preferred that the extended region image is extended to the area where an existence of the indicator does not affect the mucosal image. In this way, the influence of the existence of the indicator can be reliably eliminated.

In the case that the contour of the mucosal image is circular or polygonal, it is preferred that the extended region image is an image that forms annular shape surroundings outer periphery of the mucosal image. In this way, the image shapes of the learning image data are unified, and the effects of differences in the image shapes can be reduced.

The color conversion correction can be performed, based on an average and a dispersion of pixels of the mucosal image, to convert the mask image and the extended region image into a pattern image of a color that does not affect the mucosal image.

The color tone correction can be performed, using the most frequent values of hue, saturation and value of the mucosal image as a reference, on the hue, saturation and value (brightness) of the whole mucosal images so that their distribution is not biased.

When diagnosing endoscopic images of a bladder of a patient with Hunner-type interstitial cystitis by using the learning model learned by using the learning image data created by the method of the present invention, the diagnostic accuracy of Hunner-type interstitial cystitis can be significantly improved compared to conventional methods.

The present invention can be grasped as expanded learning image data creating system for creating the expanded learning image data with a data processor, based on an endoscopic image in which a mask image exists outside a mucosal image. In the case mentioned above, it is preferred that the data processor comprises extension processing section that creates extension processed learning image data that has an extended region image of the mask image which is made by a mask extension process that defines the extended region image with a region which is adjacent to the mask image and where the mucosal image is distorted due to the influence of a lens of an endoscope for observing mucosal, color replacement processing section that performs color conversion correction on the mask image of the extension processed learning image data to create mask color replaced learning image data, and color tone correction processing section that corrects the color tone of the mucosal image as the difference in the color tone of the mucosal image in the mask color replaced learning image data is not affected to create mucosal color tone corrected learning image data.

In addition, the data processor further comprises expansion processing section that creates expanded learning image data by image expansion technologies, using the mucosal color tone corrected learning image data.

BRIEF DESCRIPTION OF DRAWINGS

Fig. 1 is a block diagram illustrating the configuration of the main parts of an image diagnostic system equipped with an expanded learning image data creating system which carries out a learning image data creating method of the present invention.

Fig. 2 is a flowchart illustrating an outline of an algorithm of a program for automatically creating learning image data by using a computer in Fig. 1.

Figs. 3A, 3B and 3C are schematic diagrams illustrating image processing states.

Figs. 4A, 4B and 4C are schematic diagrams illustrating image processing states.

Fig. 5 is a flowchart illustrating an outline of an algorithm of a program for color conversion correction.

Fig. 6 is a flowchart illustrating an outline of an algorithm of a program for color tone correction.

Figs. 7A, 7B, 7C and 7D are diagrams illustrating examples of actual images obtained by image data processing.

Fig. 8A is a diagram illustrating the results when the AUC value is used as an evaluation indicator for each learning model, and Fig. 8B is a diagram illustrating the results when the ROC curves are obtained as the evaluation indicators.

DESCRIPTION OF EMBODIMENTS

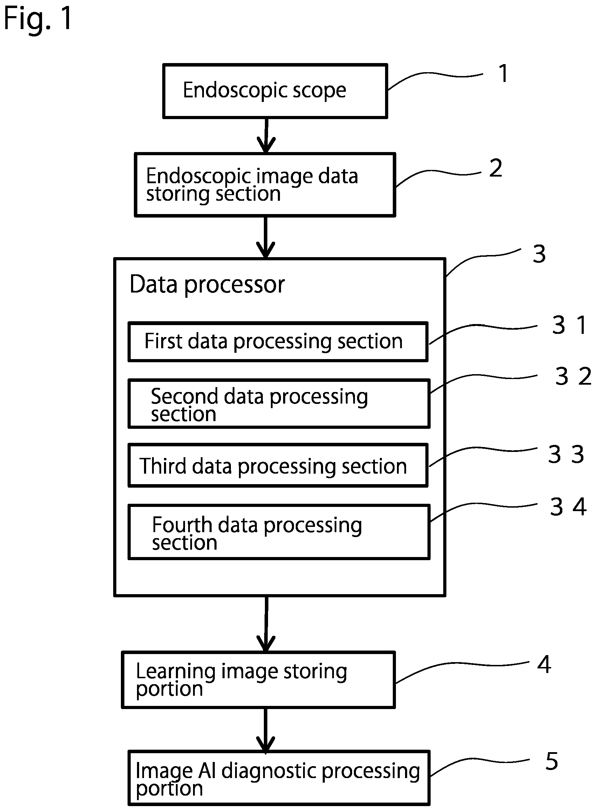

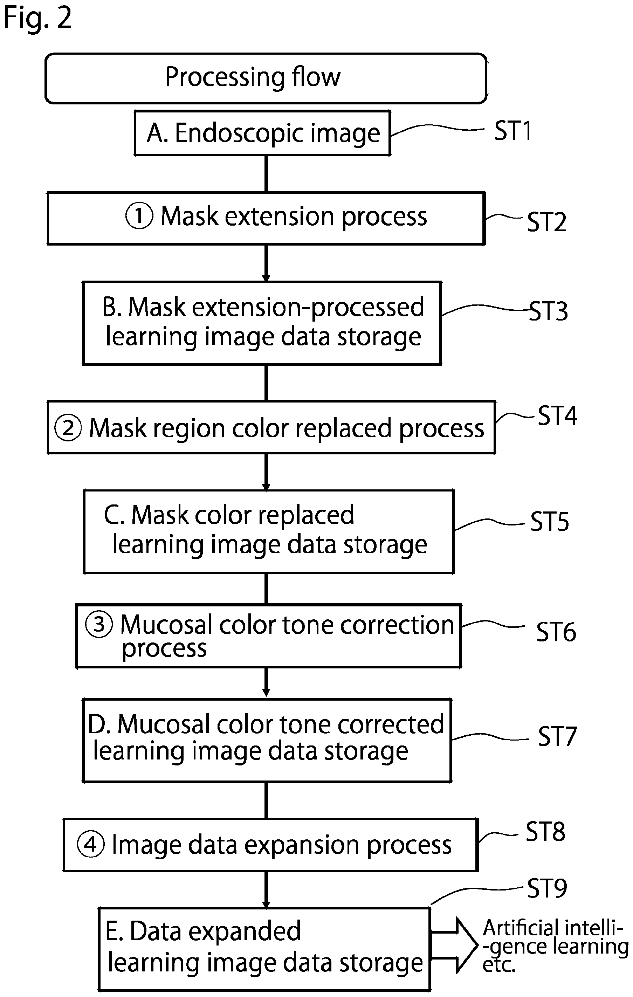

Examples of a present embodiment of the present invention will be explained in detail with reference to the drawings as follows. Fig. 1 is a block diagram illustrating the configuration of the main parts of an image diagnostic system equipped with an expanded learning image data creating system which carries out a learning image data creating method of the present invention. Fig. 2 is a flowchart illustrating an outline of an algorithm of a program for automatically creating learning image data by using a computer in the system illustrated in Fig. 1. The system stores endoscopic image data photographed by an endoscopic scope 1 in an endoscopic image data storing section 2 as illustrated in Fig. 1. The endoscopic image data stored in the endoscopic image data storing section 2 performs data processing by a data processor 3.

The data processor 3 creates expanded learning image data using an endoscopic image in which a mask image exists outside a mucosal image as learning image data. The expanded learning image data created by the data processor 3 is stored in learning image storing section 4, and the expanded learning image data is utilized for AI diagnosis in an image AI diagnostic processing portion 5.

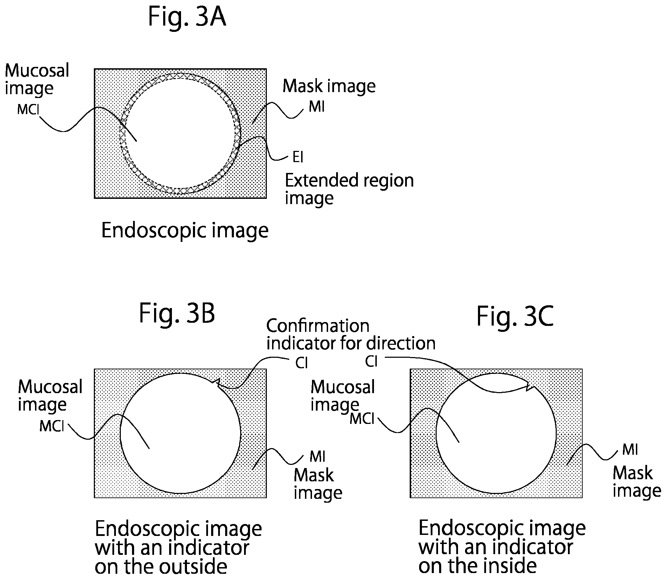

First data processing section 31, second data processing section 32, third data processing section 33 and fourth data processing section 34 are implemented in the data processor 3 by installing a program in the data processor 3. The first data processing section 31 (extension processing section) creates an extension processed learning image that has an extended region image EI of the mask image MI which is made by a mask extension process that defines the extended region image with a region which is adjacent to a mask image MI and where a mucosal image MCI is distorted due to an influence of a lens of the endoscopic scope 1 for observing mucosal as illustrated in a schematic diagram in Fig. 3A (steps ST2 and ST3 both illustrated in Fig. 2). The mask image MI includes a confirmation indicator for direction CI that confirms the up and down direction of the endoscopic image as illustrated in Fig. 3B and Fig. 3C. Then, when the mask extension process is performed, the extended region image EI may be extended to the area where an existence of the confirmation indicator for direction CI does not affect the mucosal image MCI. For example, in the case that there is an inward-facing confirmation indicator for direction CI as illustrated in Fig. 3C, the extended region image EI may be extended at least to the extent that the confirmation indicator for direction CI can be hidden. In addition, in the case that there is an outward-facing confirmation indicator for direction CI as illustrated in Fig. 3B, the outward-facing confirmation indicator for direction CI can be hidden and the inward-facing extended region area EI may be extended to the extent of a radial dimension close to a height dimension of the confirmation indicator for direction CI. In the present embodiment, the amount of the extended region image EI may be previously decided based on the presence or absence of the confirmation indicator for direction CI, known dimensional information of the confirmation indicator for direction CI, known radial dimension information of the mask, or the like, but the amount of the extended region image EI can be decided based on the arithmetic expression for the extension amount being proportional to the inner diameter of the mask calculated by experiments in advance.

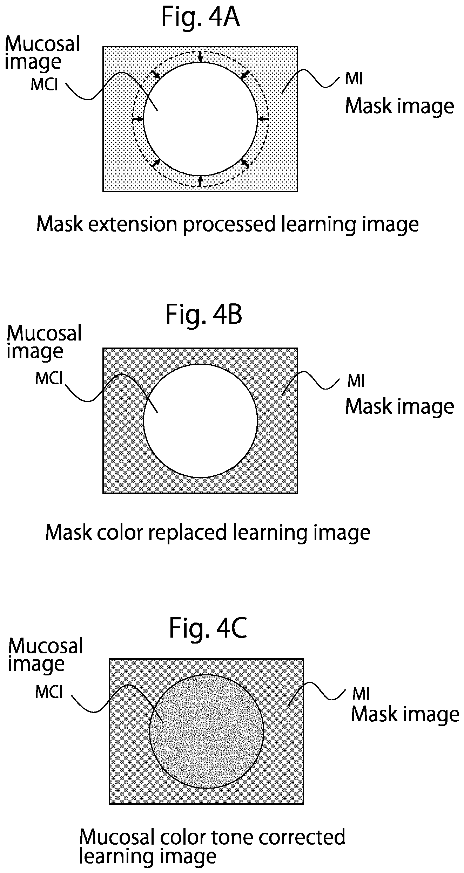

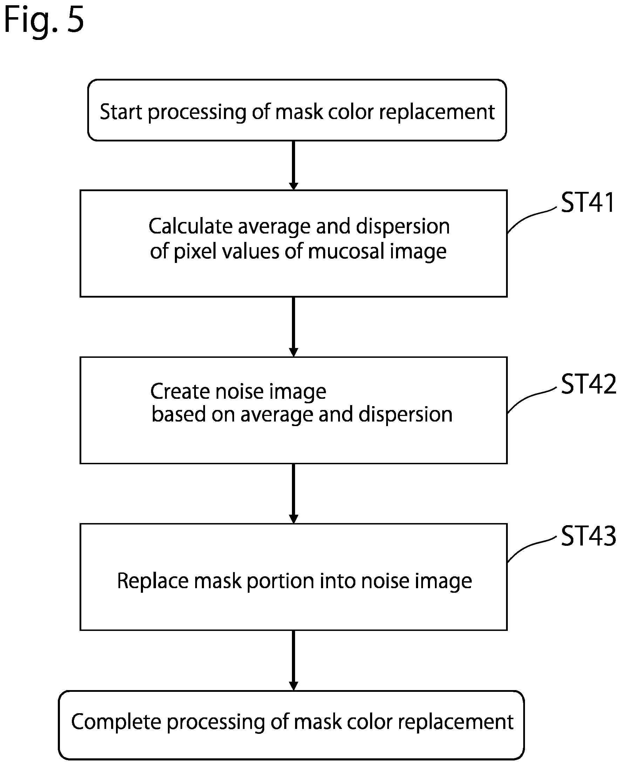

Second data processing section (color replacement processing section) 32 creates mask color replaced learning image data by performing color conversion correction on a mask image. The second data processing section 32 obtains an average and a standard deviation of RGB of the mucosal image, generates a uniformly distributed pattern image according to the average and the standard deviation of RGB, and replaces the mask image with the pattern image. The pattern image is a so-called image of a sandstorm-like pattern in which colors are distributed within a range of the standard deviation centered on the average of RGB. In particular, as illustrated in Figs. 4A and 4B, the color conversion correction replaces the mask image MI and the extended region image EI to the pattern image with the color which does not make the mucosal image MCI cause influence. The color conversion correction is performed according to the flowchart of the program as illustrated in Fig. 5. First of all, the average and the dispersion of pixel values of the mucosal image are calculated (step ST41). In the calculation, when the shading value of a pixel in the mucosal image MCI is $x_j$ (j = 1, 2, ... M), the average $\mu$ and the standard deviation $\sigma$ of the shading value can be expressed as the following formula 1 and formula 2.

[Formula 1]

$$\mu = \frac{1}{M}\sum_{j=1}^{M} x_j$$

[Formula 2]

$$\sigma = \sqrt{\frac{1}{M}\sum_{j=1}^{M}\left(x_j - \mu\right)^2}$$

Second of all, a pattern image is created based on the average and the dispersion (step ST42). In particular, the pixels $g_i$ (i = 1, 2, ..., N) in the pattern image $I_{texture}$ are filled using the uniformly distributed random numbers within the following range defined by the average $\mu$ and the standard deviation $\sigma$ each calculated.

[Formula 3]

$$\mu - \sigma \leq g_i < \mu + \sigma$$

Finally, the mask portion is replaced to pixel images. The replacement is performed by the pixel value of the pattern image $L_{texture}$ that has created the pixel of the region of the mask image MI. The mask color replacement process is completed by the above-mentioned performance. Note that the process mentioned above is an example, and the present invention is not limited to the process.

Next, third data processing section (color tone correction processing section) 33 creates a mucosal color tone corrected learning image by performing color tone correction of the mucosal image as the differences in the color tone of the mucosal image in the mask color replaced learning image do not affect the learning results, as illustrated diagrammatically in Fig. 4C. Specifically, a hue shift and a flattening process of saturation and value (brightness) are performed. First, the mucosal image comprising a mask processed RGB image is converted into an HSV image, and the most frequent value of the hue is sifted to the center. The shift of the most frequent value of the hue to the center means that, if an image is entirely reddish, the color distribution is shifted so that the colors are distributed around the red areas. In addition, adaptive histogram equalization is performed on the saturation and the value of the hue shifted image. The adaptive histogram equalization means that the saturation and the value of the image are processed as distributed evenly. According to the process mentioned above, even if the color tone of the mucosal surface is changed due to the type of light source used by the endoscopic device, it would be possible to create a similar color tone by flattening the bias of the saturation and the value, and to make it easier to see the condition of the mucosal surface.

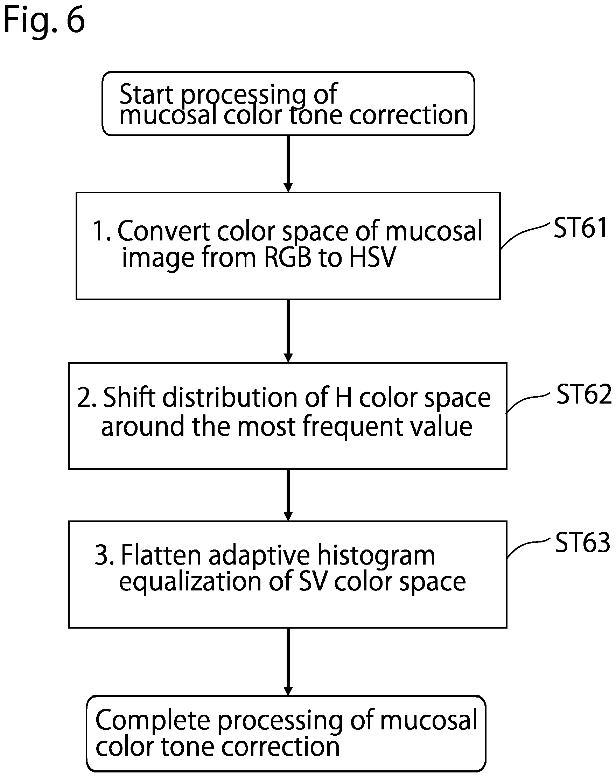

The color tone correction mentioned above is performed according to the flowchart illustrated in Fig. 6 (steps ST6, ST7 illustrated in Fig. 2). First, the color space of the mucosal image is converted from RGB into HSV (step ST61). When the pixel values of red, green and blue that are colors of pixels in the mucosal image MCI are $x_j = (R_j, G_j, B_j)$ (j = 1, ..., M), the pixel values are converted to HSV color space (H: Hue, S: Saturation and V: Value (Brightness)). Here, $MAX_j$ means the maximum value of RGB colors in pixels respectively, and $MIN_j$ means the minimum value of RGB colors in pixels respectively.

[Formula 4]

$$H_j = \begin{cases} 60° \times \dfrac{G_j - B_j}{MAX_j - MIN_j} + 0° \; if \; MAX_j = R_j \\[2mm] 60° \times \dfrac{B_j - R_j}{MAX_j - MIN_j} + 120° \; if \; MAX_j = G_j \\[2mm] 60° \times \dfrac{R_j - G_j}{MAX_j - MIN_j} + 240°, \; if \; MAX_j = B_j \end{cases}$$

$$S_j = \frac{MAX_j - MIN_j}{MAX_j}$$

$$V_j = MAX_j$$

Next, the distribution of H color space is shifted around the most frequent value (step ST62). Specifically, each pixel of the HSV color spaces is expressed as the tint value of L level, and when the number of pixels of the level i (i = 0, 1, ..., L) denotes $n_i$, the pixel distribution in the Hue color space is shifted in parallel according to the following formula, as the most frequent value level $i_{max}$ which $n_i$ is maximum is positioned in the center of the distribution.

[Formula 5]

$$H'_j = \left(H_j + 180° - i_{max,hue}\right) \; mod \; 360°$$

Then lastly, the adaptive histogram equalization is performed on the SV color space. That is, when the total number of pixels for the mucosal image is M = $n_0$ + $n_1$ + ... + $n_L$, and the maximum values of the number of pixels in the Saturation space and Value color space are $S_{max}$ and $V_{max}$, respectively, the pixel distribution of each color space is flattened according to the following formula.

[Formula 6]

$$S'_j = \frac{S_{max}}{N} \sum_{i=0}^{S_j} n_i \qquad\qquad V'_j = \frac{V_{max}}{N} \sum_{i=0}^{V_j} n_i$$

As explained above, if color tone of the mucosal image is corrected, the color tone difference of images can be corrected corresponding to the type of endoscopes to be used. Note that the color tone correction can be performed before the above-mentioned color conversion correction. Even if a learning model is created as learning image data is mucosal color tone corrected learning image data, the learning accuracy of the learning model can be improved.

In addition, fourth data processing section (expansion processing section) 34 creates expanded learning image data by image expansion technologies, using the mucosal color tone corrected learning image data (step ST8 illustrated in Fig. 2). The present embodiment uses expanded learning image data as learning image data. Thereby, the learning accuracy of the learning model can be improved without compensating for the quality of the learning data with the amount of training data. The image expansion technologies mentioned above may be well-known data expansion technologies. As a preferable expansion technology, rotating, flipping, blurring, and translating or the like of images each mounted on machine learning frameworks such as Keras, PyTorch, etc. can be used.

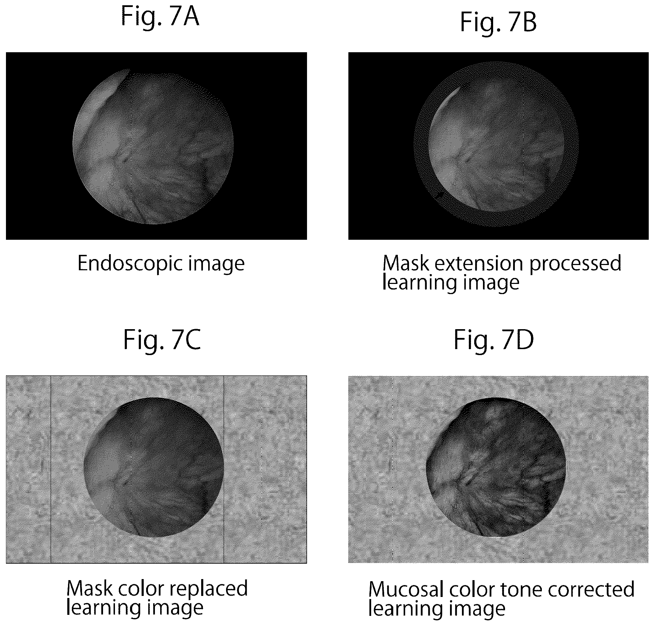

Figs. 7A, 7B, 7C and 7D are examples of actual images performed by the above-mentioned image data process. Fig. 7A is an endoscope image, Fig. 7B is a mask expanded learning image, Fig. 7C is a mask color replaced learning image and Fig. 7D is a mucosal color tone corrected image. Note that an expansion learning image data may be created by image expansion technology using the mucosal color tone corrected learning image data, and the expansion learning image data may be the learning image data. Also, the learning model may be created as the mucosal color tone corrected learning image data as the learning image data.

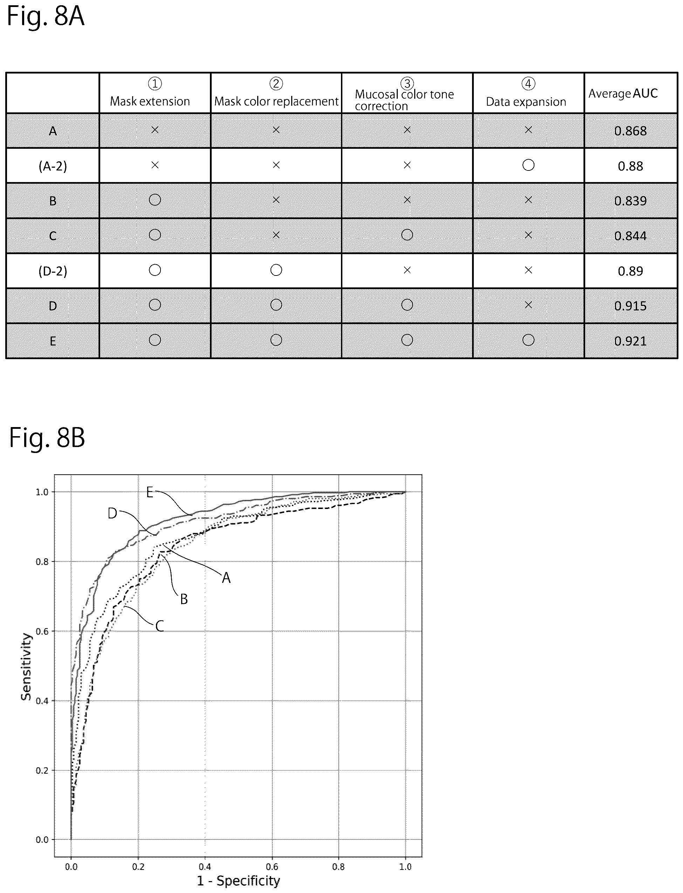

The inventors of the present invention have performed an accuracy assessment for the learning models (A, A-2, B, C, D, D-2 and E) created by using the image data, as the learning image data, obtained by combining the above-mentioned "mask extension", "mask color replacement", "mucosal color tone correction" and "data expansion" based on endoscopic image data being very few cases as Hunner-type interstitial cystitis. Fig. 8A is a diagram illustrating the results when the AUC (Area Under the Curve) value is used as an evaluation indicator for each learning model, and Fig. 8B is a diagram illustrating the results when the ROC (Receiver Operating Characteristic) curve is obtained as the evaluation indicator. The image processing technology used for the comparison mentioned above is the combination of the above-mentioned "mask extension", "mask color replacement", "mucosal color tone correction" and "data expansion." The learning model A illustrated in Fig. 8 is a learning model created by using endoscopic image data without any image process, as learning image data, and the average AUC value was 0.868. The learning model A-2 is a learning model created by using endoscopic image data itself and expanded learning image data from the endoscopic image data, and the average AUC value was 0.88. The learning model B is a learning model created by using extended learning image data obtained by mask extension, as learning image data, and the average AUC value was 0.839. The learning model C is a learning model created by mucosal color tone correction of extended learning image data obtained by mask extension, as learning image data, and the average AUC value was 0.844. The learning model D-2 is a learning model created by mask color replacement of extended learning image data obtained by mask extension, as learning image data, and the average AUC value was denoted. The learning model D is a learning model created by mask color replacement and mucosal color tone correction of extended learning image data obtained by mask extension, as learning image data, and the average AUC value was 0.915. The learning model E is a learning model created by an expansion technology that expands extended learning image data obtained by mask extension, as the expansion learning image data is learning image data, and the average AUC value was 0.921.

As the result of the average AUC values obtained above, the average AUC value was 0.9 or more for the learning model D or the learning model E so that it is confirmed to obtain a higher learning accuracy for the learning model D or the learning model E.

Furthermore, ROC curves illustrated in Fig. 8B illustrate the ROC curves for the learning model A, the learning model B, the learning model C, the learning model D and the learning model E. The horizontal axis of the ROC curve is "1-specificity" and the vertical axis of the ROC is "sensitivity." Ideal learning models in terms of performance are the learning models in which the sensitivity and specificity of the ROC curve approach the upper left corner. Therefore, it would be defined that the learning model D and the learning model E have higher learning accuracies from the point of view of the ROC curves illustrated in Fig. 8B. As the result explored above, it was confirmed that by using the learning model D or the learning model E, a learning accuracy of the learning model can be improved without compensating for the quantity of learning data with the amount of learning data.

Note that it has been confirmed that the diagnostic accuracy of patients with Hunner-type interstitial cystitis is significantly higher than the conventional method when making diagnoses based on the endoscopic images of the patients with Hunner-type interstitial cystitis by using the learning model E.

INDUSTRIAL APPLICABILITY

According to the present invention, few noise is included in the learning image data so that it is possible to obtain the higher learning accuracy of the learning model.

EXPLANATION OF REFERENCE NUMERALS

1: endoscopic scope, 2: endoscopic image data storing section, 3: data processor, 31: first data processing section (extension processing section), 32: second data processing section (color replacement processing section), 33: third data processing section (color tone correction processing section), 34: fourth data processing section (expansion processing section), 4: learning image storing portion, 5: image AI diagnostic processing portion.

**Claims**

1. A learning image data creating method for creating learning image data used to create a learning model, based on an endoscopic image in which a mask image (MI) exists outside a mucosal image (MCI), **characterized in that** the method comprises steps of:

   creating extension processed learning image data that has an extended region image (EI) of the mask image (MI) which is made by a mask extension process (ST2) that defines the extended region image with a region which is adjacent to the mask image (MI) and where the mucosal image (MCI) is distorted due to the influence of a lens of an endoscope for observing mucosal,
   creating the learning image data based on the extension processed learning image data,
   creating mask color replaced learning image data by performing color conversion correction (ST4) on the mask image (MI) and the extended region image (EI) of the extension processed learning image data,
   creating mucosal color tone corrected learning image data by performing the color tone correction (ST6) of the mucosal image (MCI) as the difference in the color tone of the mucosal image (MCI) in the mask color replaced learning image data is not affected, and
   defining the mucosal color tone corrected learning image data as the learning image data.

2. The learning image data creating method according to claim 1, further comprises the following steps of:

   creating expanded learning image data by image expansion technologies (ST8), using the mucosal color tone corrected learning image data, and
   defining the expanded learning image data as the learning image data.

3. The learning image data creating method according to claim 1, wherein:
   the mask image includes an indicator (CI) that confirms the up and down direction of the endoscopic image, and the extended region image (EI) is extended beyond an area where an existence of the indicator (CI) affects the mucosal image.

4. The learning image data creating method according to claim 2, wherein:
   the mask image includes an indicator (CI) that confirms the up and down direction of the endoscopic image, and the extended region image (EI) is extended beyond an area where an existence of the indicator (CI) affects the mucosal image.

5. The learning image data creating method according to claim 1, wherein:
   the contour of the mucosal image is circular or polygonal, and the extended region image (EI) is an image that forms annular shape surroundings outer periphery of the mucosal image (MCI).

6. The learning image data creating method according to claim 2, wherein:
   the contour of the mucosal image is circular or polygonal, and the extended region image (EI) is an image that forms annular shape surroundings outer periphery of the mucosal image (MCI).

7. The learning image data creating method according to claim 1, wherein:
   the color conversion correction is performed, based on an average and a dispersion of pixels of the mucosal image (MCI), to convert the mask image (MI) and the extended region image (EI) into a pattern image of a color that does not affect the mucosal image (MCI).

8. The learning image data creating method according to claim 1, wherein:
   the color tone correction is performed, using the most frequent values of hue, saturation and value of the mucosal image (MCI) as a reference, on the hue, saturation and value of the whole mucosal image (MCI) so that their distribution is not biased.

9. A method for diagnosing Hunner-type interstitial cystitis comprises diagnosing endoscopic images of a bladder of a patient with Hunner-type interstitial cystitis by using the learning model learned by the using learning image data created by the method according to any one of claims 1 to 8.

10. A learning image data creating system for creating learning image data used to create a learning model with a data processor (3), based on an endoscopic image in which a mask image (MI) exists outside a mucosal image (MCI), wherein:

the data processor (3) comprises;
extension processing section (31) that creates extension processed learning image data that has an extended region image (EI) of the mask image (MI) which is made by a mask extension process that defines the extended region image (EI) with a region which is adjacent to the mask image (MI) and where the mucosal image (MCI) is distorted due to the influence of a lens of an endoscope for observing mucosal,
color replacement processing section (32) that performs color conversion correction on the mask image (MI) of the extension processed learning image data to create mask color replaced learning image data, and
color tone correction processing section (33) that corrects the color tone of the mucosal image (MCI) as the difference in the color tone of the mucosal image (MCI) in the mask color replaced learning image data is not affected to create mucosal color tone corrected learning image data,
wherein the mucosal color tone corrected learning image data is defined as the learning image data.

11. The learning image data creating system according to claim 10, wherein:
the data processor (3) further comprises expansion processing section (34) that creates creating expanded learning image data by image expansion technologies, using the mucosal color tone corrected learning image data.

12. The learning image data creating system according to claim 10, wherein:

the mask image (MI) includes an indicator (CI) that confirms the up and down direction of the endoscopic image, and
the extension processing section (31) that extends the extended region image (EI) beyond the area where an existence of the indicator (CI) affects the mucosal image (MCI).

13. The learning image data creating system according to claim 10, wherein:
the contour of the mucosal image (MCI) is circular or polygonal, and the extended region image (EI) is an image that forms annular shape surroundings outer periphery of the mucosal image (MCI).

14. The learning image data creating system according to claim 10, wherein:
the color replacement processing section (32) is so constructed that color correction is performed based on the most frequent value of hue, saturation and value of the mucosal image (MCI) as a reference, on the hue, saturation and value of the whole mucosal image (MCI) so that their distribution is not biased.

Fig. 1

```
Endoscopic scope                        1

        ↓

Endoscopic image data                   2
storing section

        ↓

Data processor                          3

    First data processing section       3 1

    Second data processing              3 2
    section

    Third data processing section       3 3

    Fourth data processing              3 4
    section

        ↓

Learning image storing                  4
portion

        ↓

Image AI diagnostic processing          5
portion
```

Fig. 2

```
┌─────────────────────────────────────┐
│           Processing flow            │
└─────────────────────────────────────┘
        ┌─────────────────────┐
        │ A. Endoscopic image │ ──── ST1
        └─────────────────────┘
                  │
                  ▼
  ┌─────────────────────────────────────┐
  │ ① Mask extension process            │ ──── ST2
  └─────────────────────────────────────┘
                  │
                  ▼
  ┌─────────────────────────────────────┐
  │ B. Mask extension-processed         │ ──── ST3
  │    learning image data storage      │
  └─────────────────────────────────────┘
                  │
                  ▼
  ┌─────────────────────────────────────┐
  │ ② Mask region color replaced process│ ──── ST4
  └─────────────────────────────────────┘
                  │
                  ▼
  ┌─────────────────────────────────────┐
  │ C. Mask color replaced              │ ──── ST5
  │    learning image data storage      │
  └─────────────────────────────────────┘
                  │
                  ▼
  ┌─────────────────────────────────────┐
  │ ③ Mucosal color tone correction     │ ──── ST6
  │    process                          │
  └─────────────────────────────────────┘
                  │
                  ▼
  ┌─────────────────────────────────────┐
  │ D. Mucosal color tone corrected     │ ──── ST7
  │    learning image data storage      │
  └─────────────────────────────────────┘
                  │
                  ▼
  ┌─────────────────────────────────────┐
  │ ④ Image data expansion process      │ ──── ST8
  └─────────────────────────────────────┘
                  │                        ──── ST9
                  ▼
  ┌─────────────────────────────────────┐    Artificial intelli-
  │ E. Data expanded                    │ ⇒  -gence learning
  │    learning image data storage      │    etc.
  └─────────────────────────────────────┘
```

# Fig. 3A

Mucosal image
MCI

Mask image
MI

EI
Extended region image

Endoscopic image

# Fig. 3B

Confirmation indicator for direction
CI

Mucosal image
MCI

MI
Mask image

Endoscopic image with an indicator on the outside

# Fig. 3C

CI

Mucosal image
MCI

MI
Mask image

Endoscopic image with an indicator on the inside

## Fig. 4A

Mucosal image MCI

MI Mask image

Mask extension processed learning image

## Fig. 4B

Mucosal image MCI

MI Mask image

Mask color replaced learning image

## Fig. 4C

Mucosal image MCI

MI Mask image

Mucosal color tone corrected learning image

# Fig. 5

```
┌─────────────────────────────────────────────┐
│   Start processing of mask color replacement  │
└─────────────────────────────────────────────┘
                      │
                      ▼
┌─────────────────────────────────────────────┐        ST41
│      Calculate average and dispersion         │
│       of pixel values of mucosal image        │
└─────────────────────────────────────────────┘
                      │
                      ▼
┌─────────────────────────────────────────────┐        ST42
│           Create noise image                  │
│      based on average and dispersion          │
└─────────────────────────────────────────────┘
                      │
                      ▼
┌─────────────────────────────────────────────┐        ST43
│      Replace mask portion into noise image    │
└─────────────────────────────────────────────┘
                      │
                      ▼
┌─────────────────────────────────────────────┐
│  Complete processing of mask color replacement │
└─────────────────────────────────────────────┘
```

# Fig. 6

Start processing of
mucosal color tone correction

↓

1. Convert color space of mucosal
image from RGB to HSV — ST61

↓

2. Shift distribution of H color space
around the most frequent value — ST62

↓

3. Flatten adaptive histogram
equalization of SV color space — ST63

↓

Complete processing of mucosal
color tone correction

Fig. 7A

Endoscopic image

Fig. 7B

Mask extension processed
learning image

Fig. 7C

Mask color replaced
learning image

Fig. 7D

Mucosal color tone corrected
learning image

## Fig. 8A

| | ①<br>Mask extension | ②<br>Mask color replacement | ③<br>Mucosal color tone correction | ④<br>Data expansion | Average AUC |
|---|---|---|---|---|---|
| A | × | × | × | × | 0.868 |
| (A-2) | × | × | × | ○ | 0.88 |
| B | ○ | × | × | × | 0.839 |
| C | ○ | × | ○ | × | 0.844 |
| (D-2) | ○ | ○ | × | × | 0.89 |
| D | ○ | ○ | ○ | × | 0.915 |
| E | ○ | ○ | ○ | ○ | 0.921 |

## Fig. 8B

## INTERNATIONAL SEARCH REPORT

| International application No. |
|---|
| **PCT/JP2023/022550** |

**A.    CLASSIFICATION OF SUBJECT MATTER**

*A61B 1/045*(2006.01)i; *G06T 1/00*(2006.01)i
FI:    A61B1/045 614; A61B1/045 618; G06T1/00 290Z

According to International Patent Classification (IPC) or to both national classification and IPC

**B.    FIELDS SEARCHED**

Minimum documentation searched (classification system followed by classification symbols)

A61B1/00-1/32; G06T1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
|---|---|---|
| A | 岩城拓弥, 人工知能によるハンナ型間質性膀胱炎の内視鏡診断支援システムの開発, 排尿障害プラクティス, June 2022, vol. 30, no. 1, pp. 72-75, ISSN:0919-5750, in particular, see p. 75, left column<br>entire text, (IWAKI, Takuya. Hainyo Shogai Practice.), non-official translation (Development of an endoscopic diagnosis assistance system for Hunner-type interstitial cystitis using artificial intelligence) | 1-14 |
| A | WO 2022/114088 A1 (TOMO CO LTD) 02 June 2022 (2022-06-02)<br>entire text, all drawings | 1-14 |
| A | JP 06-254044 A (FUJI PHOTO OPTICAL CO LTD) 13 September 1994 (1994-09-13)<br>entire text, all drawings | 1-14 |

☐ Further documents are listed in the continuation of Box C.       ☑ See patent family annex.

| | |
|---|---|
| *    Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **10 August 2023** | **22 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/022550**

| Patent document cited in search report | | | Publication date (day/month/year) | Patent family member(s) | | | Publication date (day/month/year) |
|---|---|---|---|---|---|---|---|
| WO | 2022/114088 | A1 | 02 June 2022 | US 2022/0392063 A1 entire text, all drawings TW 202236297 A | | | |
| JP | 06-254044 | A | 13 September 1994 | (Family: none) | | | |

Form PCT/ISA/210 (patent family annex) (January 2015)

**REFERENCES CITED IN THE DESCRIPTION**

**Patent documents cited in the description**

- JP 2020141995 A

- JP 3720617 B